Europäisches Patentamt

European Patent Office

Office européen des brevets

(11) Publication number: **0 472 963 A2**

# EUROPEAN PATENT APPLICATION

(21) Application number: **91113213.2**

(22) Date of filing: **06.08.91**

(51) Int. Cl.5: **H01L 33/00**, H01L 31/167, A61B 5/024

(30) Priority: **27.08.90 JP 89655/90 U**
**27.08.90 JP 89656/90 U**

(43) Date of publication of application:
**04.03.92 Bulletin 92/10**

(84) Designated Contracting States:
**DE FR GB IT**

(71) Applicant: **SUMITOMO ELECTRIC INDUSTRIES, LIMITED**
**5-33, Kitahama 4-chome Chuo-ku Osaka(JP)**

(72) Inventor: **Osawa, Masahiko, c/o Osaka Works of Sumitomo**
**Elec. Works, Ltd., 1-3, Shimaya 1-chome Konohana-ku, Osaka(JP)**

(74) Representative: **Herrmann-Trentepohl, Werner, Dipl.-Ing. et al**
**Kirschner, Grosse, Bockhorni Forstenrieder Allee 59**
**W-8000 München 71(DE)**

(54) **Light emitting diode for photosensor and photosensor using such light emitting diode.**

(57) An improved light emitting diode for a photosensor which enables correction of a change in the amount of light from a light emitting element (LED₂) in accordance with a change in ambient temperature is disclosed. The light emitting diode for a photosensor includes a substrate (12) and a light emitting element (LED₂) provided on the substrate (12). A light receiving element (PD) which receives the light emitted from the light emitting element (LED₂) thereby to detect a change in the amount of the light from the light emitting element (LED₂) in accordance with a change in ambient temperature is disposed on the substrate (12) and near the light emitting element (LED₂). A first transparent layer (21) is provided on the substrate (12) to cover the light emitting element (LED₂) and the light receiving element (PD). A second transparent layer (22) is provided on the first transparent layer (21). A very thin air layer is interposed between the first and second transparent layer (21, 22).

FIG.6

## BACKGROUND OF THE INVENTION

### Field of the Invention

The present invention relates generally to light emitting diodes for photosensors and, more particularly, to an improved light emitting diode for a photosensor which enables correction of changes in the amount of light from a light emitting element in accordance with changes in ambient temperature. The invention further relates to a photosensor using such a light emitting diode.

### Description of the Background Art

Conventionally, an apparatus has been known which transmits light from one side to the other side of a finger tip and detects changes in the transmittance (reflectance) of the transmitted light, thereby to examine the amount of blood flowing in the finger, process a resultant detection signal and then evaluate pulsation, blood pressure and the like by calculation. One example of a photosensor for use in such an apparatus is disclosed in Japanese Utility Model Laying-Open No. 60-158803.

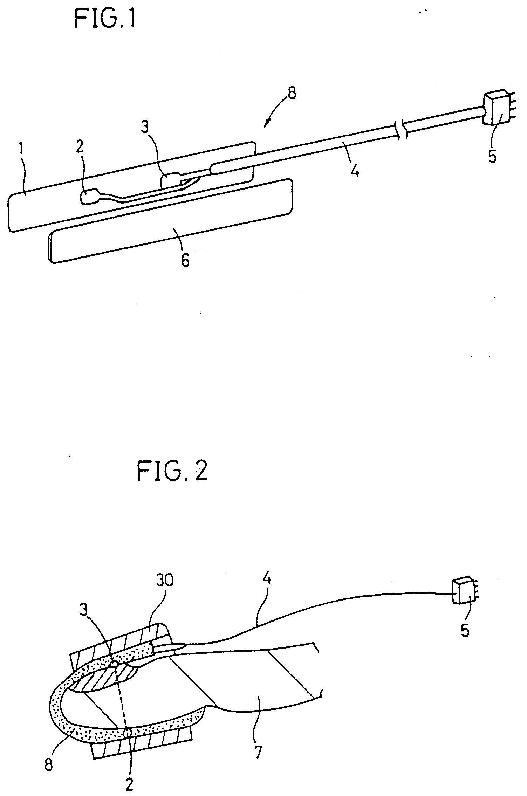

Fig. 1 is a perspective view of a conventional photosensor, and Fig. 2 is a cross-sectional view of the photosensor of Fig. 1 attached to a finger.

Description will now be made on a conventional photosensor 8 by reference to Fig. 1. Photosensor 8 serves to transmit light from one side to the other side of the finger, so as to detect changes in the transmittance of the light. A light emitting element 2 and a light receiving element 3 are disposed with a predetermined spacing therebetween corresponding to the size of the finger, on an easily bendable film substrate 1. A transparent and easily bendable transparent film 6 is placed or attached to film substrate 1 to cover light emitting element 2 and light receiving element 3.

Description will now be given on a method of using photosensor 8 by reference to Fig. 2. Photosensor 8 is wound around a finger 7 to interpose the tip of finger 7 between light emitting element 2 and light receiving element 3. A fixing tape 30 (e.g., a so-called magic tape) is attached around photosensor 8 wound around finger 7. Photosensor 8 is securely fixed on finger 7 by winding fixing tape 30 around photosensor 8 and overlapping the surface of one end of fixing tape 30 and the reverse surface of the other end thereof. When power is supplied from a main body of a signal processing unit (not shown) via a connecter 5 to a lead 4, light emitting element 2 emits light. The emitted light is transmitted through finger 7 and directed onto light receiving element 3. Light receiving element 3 receives the light and transmits a resultant signal via lead 4 and connector 5 to the main body of the signal processing unit. The signal processing unit detects a change in the transmittance of the light provided at this time, processes a detection signal thereof and then evaluates pulsation and a blood pressure value by calculation.

In general, a light emitting diode is used for light emitting element 2 employed in the above-described photosensor 8. The light emitting diode has, however, an undesirable property that its output power and a wavelength of light emitted from the diode vary depending on ambient temperatures. If photosensor 8 is attached to a living body, e.g., finger 7, then finger 7 becomes ischemic or hemostatic, resulting in a decrease in the body temperature of finger 7, or an increase in the body temperature thereof due to an increase in blood pressure. Thus, the ambient temperature of light emitting element 2 varies, and its output power and its measured wavelength of light also vary. However, it is desirable that the output power of light emitting element 2 and the wavelength of the light emitted from light emitting element 2 are kept constant in order to accurately measure a pulse value or a blood pressure value.

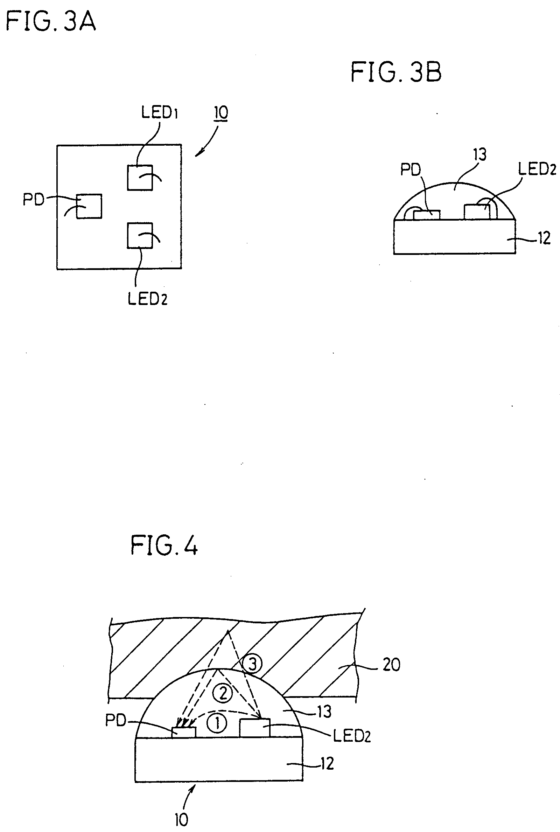

The inventors have proposed a light emitting diode shown in Figs. 3A and 3B as the one satisfying the above requirements (Japanese Patent Application No. 1-116757). Fig. 3A is a plan view of the proposed light emitting diode, and Fig. 3B is a side view of the diode of Fig. 3A. With reference to Figs. 3A and 3B, a light emitting diode 10 includes two LED chips $LED_1$ and $LED_2$ disposed on a substrate 12. A photodiode PD is provided in the vicinity of LED chips $LED_1$ and $LED_2$. Photodiode PD directly receives light emitted from LED chips $LED_1$ and $LED_2$.

Photodiode PD serves to detect a change in the amount of the light emitted from LED chips $LED_1$ and $LED_2$ in accordance with a change in ambient temperatures. A transparent epoxy resin 13 is applied onto substrate 12 to cover LED chips $LED_1$ and $LED_2$ and photodiode PD.

An operation will now be described. Photodiode PD (which is a light receiving element provided separately from the light receiving element of Fig. 1) is disposed near LED chips $LED_1$ and $LED_2$. Photodiode PD detects a change in the amount of the light from the light emitting diode in accordance with a change in ambient temperature. A current flowing through LED chips $LED_1$ and $LED_2$ is controlled so as to correct the change in the amount of the light. This makes it possible to keep output power of and a wavelength of the light emitted from LED chips $LED_1$ and $LED_2$ constant and thus to obtain accurate information as to a living body.

The light emitting diode thus structured has, however, the following disadvantages.

With reference to Fig. 4, since light emitting diode 10 is used in contact with a living body 20, there are three types of light beams: a light beam designated by the dotted line ① which is emitted from LED chip $LED_2$ and is directly incident on photodiode PD; a light beam designated by the dotted line ② which undergoes a total reflection from an inner surface of epoxy resin 13 and enters into photodiode PD; and a light beam designated by the dotted line ③ which once enters into living body 20 and is then scattered or reflected by living body 20, entering into photodiode PD. The amount of the scattered light or the reflected light denoted by the dotted line ③ is not constant. Accordingly, there occurs an error in a feedback apparatus for monitoring the amount of the light emitted from LED chips $LED_1$ and $LED_2$ to maintain a constant amount of the light. Thus, a precise feedback becomes impossible. Consequently, there is such a disadvantage that the amount of the light from the LED chips, i.e., light receiving elements cannot precisely be kept constant.

## SUMMARY OF THE INVENTION

One object of the present invention is therefore to provide an improved light emitting diode for a photosensor which is immune to influence caused by a drift of temperatures.

Another object of the present invention is to provide an improved light emitting diode for a photosensor which enables the amount of light from a light emitting element to be precisely kept constant.

A further object of the present invention is to provide an improved light emitting diode for a photosensor which enables an accurate monitor of the amount of light from a light emitting element.

A still further object of the present invention is to provide an easily manufacturable light emitting diode for a photosensor.

A still further object of the present invention is to provide a photosensor including an improved light emitting diode immune to influence caused by a drift of temperatures.

To accomplish the above objects, a light emitting diode for a photosensor in accordance with the present invention includes a substrate and a light emitting element provided on the substrate. A light receiving element for receiving light emitted from the light emitting element to detect a change in the amount of the light from the light emitting element in accordance with a change in ambient temperature is disposed on the substrate and in the vicinity of the light emitting element. A first transparent layer is formed on the substrate to cover the light emitting element and the light receiving element. A second transparent layer is formed on the first transparent layer.

Preferably, at least one of the first and second transparent layers is formed of a material which intercepts light other than the wavelength of a spectrum emitted from the light emitting element.

More preferably, an interface between the first and second transparent layers is hemispherical.

A light emitting diode for a photosensor according to another aspect of the present invention includes a substrate and a light emitting element provided on the substrate. A light receiving element which receives light emitted from the light emitting element to detect a change in the amount of the light from the light emitting element in accordance with a change in ambient temperatures is disposed on the substrate and near the light emitting element. A first transparent layer is formed on the substrate to cover the light emitting element and the light receiving element. A second transparent layer is formed on the first transparent layer. A refractive index $n_1$ of the first transparent layer and a refractive index $n_2$ of the second transparent layer satisfy the following inequality: $n_1 \geq n_2$.

A photosensor according to still another aspect of the present invention includes a light emitting diode for a photosensor having the foregoing characteristics.

In accordance with a light emitting diode for a photosensor according to the present invention, a second transparent layer is formed on a first transparent layer. Thus, a very thin air layer is formed at an interface between the first and second transparent layers in formation of the second transparent layer on the first transparent layer. A refractive index of air is smaller than those of the first and second transparent layers. Thus, the number of optical paths of the light, which is emitted from the light emitting element, then subjected to a total reflection by an inner surface of the first transparent layer and is incident on the light receiving element, increases. This makes it possible to relatively neglect the amount of the light which enters in a living body and then reflects therefrom. Consequently, the amount of the light emitted from the light emitting element, which is directly received by the light receiving element, increases.

In accordance with a light emitting diode for a photosensor according to still another aspect of the present invention, refractive index $n_1$ of the first transparent layer is larger than that $n_2$ of the second transparent layer. Thus, the number of optical paths of the light which is emitted from the light emitting element, then undergoes a total reflection at the interface between the first and second transparent layers and enters into the light receiving element increases. This makes it possible to relatively neglect the amount of the light which enters into and then reflects from the living body. Consequently, the amount of the light emitted from the light emitting element, which is directly received by the light receiving element, increases.

In accordance with a photosensor according to still another aspect of the present invention, since the photosensor includes a light emitting diode having the foregoing characteristics, a precise measurement immune to influence caused by a drift of temperatures can be made in the photosensor.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 is a perspective view of a conventional photosensor.

Fig. 2 is a cross-sectional view of the photosensor of Fig. 1 attached to a finger.

Fig. 3A is a plan view showing one example of a conventional light emitting diode; and Fig. 3B is a side view of the light emitting diode of Fig. 3A.

Fig. 4 is a diagram for use in explaining disadvantages of the conventional light emitting diode.

Fig. 5 is a side view of a light emitting diode for a photosensor according to one embodiment of the present invention.

Fig. 6 is a side view for use in explaining an operation of the light emitting diode for a photosensor according to the one embodiment.

Fig. 7 is a diagram showing a transparence property of an epoxy resin of the type which limits a wavelength band, used in the present invention.

Fig. 8 is a side view of a light emitting diode for a photosensor according to another embodiment of the present invention.

Fig. 9 is a side view for use in explaining an operation of the light emitting diode shown in Fig. 8.

DESCRIPTION OF THE PREFERRED EMBODIMENTS

Description will now be made on one embodiment of the present invention with reference to the drawings.

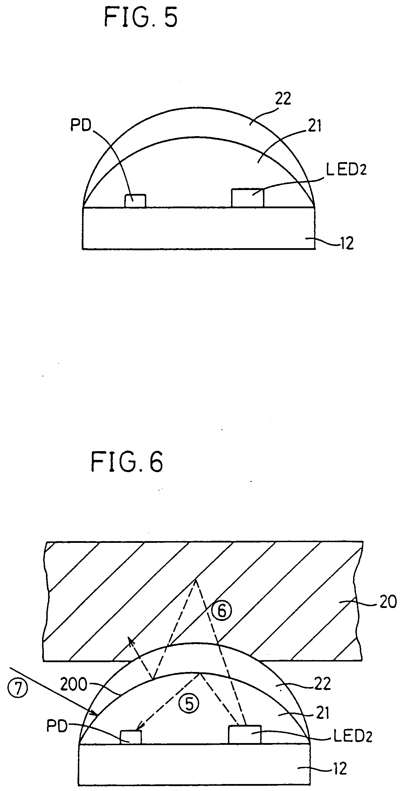

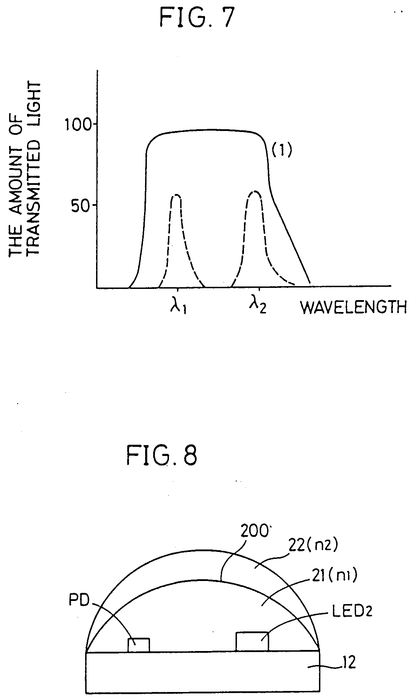

Fig. 5 is a side view of a light emitting diode for a photosensor according to the one embodiment of the present invention. The light emitting diode for a photosensor includes a substrate 12. LED chips $LED_1$ (not shown) and $LED_2$ serving as light emitting elements are disposed on substrate 12. A photodiode PD serving as a light receiving element which receives light emitted from LED chips $LED_1$ and $LED_2$, thereby to detect a change in the amount of the light from these LED chips in accordance with a change in ambient temperatures is provided on substrate 12 and in the vicinity of the LED chips. A hemispherical transparent molding material such as of a glass epoxy or the like, which is a first transparent layer 21, is formed on substrate 12 to cover LED chips $LED_1$ and $LED_2$ and photodiode PD. A hemispherical transparent molding material such as of a glass epoxy or the like, which is a second transparent layer 22, is formed on first transparent layer 21. At least one of the first and second transparent layers 21 and 22 is preferably formed of a material which intercepts light other than a wavelength of a spectrum emitted from LED chips $LED_1$ and $LED_2$. In the embodiment, first transparent layer 21 employs an epoxy resin of the type which has a transparence property shown in Fig. 7 and limits a wavelength band. Referring to Fig. 7, the curve (1) represents the transparence property of the epoxy resin for limiting the wavelength band; $\lambda_1$ designates a wavelength of a spectrum emitted from $LED_1$; and $\lambda_2$ designates a wavelength of a spectrum emitted from $LED_2$. The above-described epoxy resin for use is appropriately selected from resins, for example, Toray Hysol THL-5000A/B, HL3000 (S), EX-012/HX-021-3 and the like manufactured by Toray Hysol Co., Ltd.

A very thin air layer, not shown in Fig. 5, is formed at an interface between first and second transparent layers 21 and 22 in formation of second transparent layer 22 on first transparent layer 21. A refractive index of this air layer is smaller than those of both first and second transparent layers 21 and 22.

An operation will now be described.

With reference to Fig. 6, the light emitting diode for a photosensor is made contact with a living body 20. Since an air layer with a small refractive index is formed at an interface 200 between first and second transparent layers 21 and 22, a portion of light emitted from LED chips $LED_1$ and $LED_2$ undergoes a total reflection at interface 200 and then reaches photodiode PD, as shown by the dotted line ⑤. In the embodiment, since the amount of the light subjected to the total reflection increases, the amount of the light emitted from LED chips $LED_1$ and $LED_2$, which is directly received by photodiode PD, increases.

Light which is scattered or reflected by living body 20 as shown by the dotted line ⑥, of the light emitted from LED chips $LED_1$ and $LED_2$ and then entering into living body 20, is reflected at interface 200 between first and second transparent layers 21 and 22 and hence does not reach photodiode PD. In addition, since first transparent layer 21 employs the epoxy resin, which has the transparence property shown in Fig. 7 and limits a wavelength band, other light designated by the solid line ⑦ than the wavelength of the light emitted from LED chips LED$_1$ and LED$_2$ cannot be incident on first transparent layer 21 and are thus intercepted at interface 200.

Since the light emitting diode for a photosensor according to the embodiment is thus structured, the amount of the light which is emitted from the LED chips and is directly incident on the photodiode increases, thereby enabling a neglect of the amount of the light which is reflected from the living body and enters into the photodiode. This enables a precise feedback and consequently a precise measurement immune to influence caused by a drift of temperatures.

While such a case is illustrated that an epoxy resin is used for first and second transparent layers 21 and 22 in the foregoing embodiment, the present invention is not limited to this, and any transparent body, for example, glass or elastomer may also be employed.

While no specific explanation has been given on the thickness of the air layer, the air layer may have a thickness which is naturally grown in molding of the second transparent layer on the first transparent layer. In further detail, the thickness of the air layer may merely be larger than the wavelength of the light emitted from the LED chips.

Moreover, while such an example that first and second transparent layers 21 and 22 are hemispherical is given in the foregoing embodiment, the present invention is not limited to this. The formation of transparent layers 21 and 22 in a hemispherical shape provides the advantage of facilitating the manufacture of such transparent layers.

Fig. 8 is a side view of a light emitting diode for a photosensor according to another embodiment of the present invention. The light emitting diode for a photosensor includes a substrate 12. LED chips LED$_1$ (not shown) and LED$_2$ serving as light emitting elements are provided on substrate 12. A photodiode PD which receives light emitted from LED chips LED$_1$ and LED$_2$ to thereby detect a change in the amount of the light from the LED chips in accordance with a change in ambient temperature is provided on substrate 12 and near the LED chips. A hemispherical transparent molding material such as of a glass epoxy or the like, which is a first transparent layer 21, is formed on substrate 12 to cover LED chips LED$_1$ and LED$_2$ and photodiode PD. A hemispherical transparent molding material such as of a glass epoxy or the like, which is a second transparent layer 22, is formed on first transparent layer 21. A refractive index n$_1$ of the first transparent layer is larger than a refractive index n$_2$ of the second transparent layer. The above-described clear epoxy resin for use is appropriately selected from resins, for example, Toray Hysol THL-5000A/B, HL3000 (S) and EX-012/HX-021-3 manufactured by Toray Hysol Co., Ltd. Adjustment of the refractive indexes is made by changing compositions of those resins or by changing mixing ratios of the resins. For a manufacture method, a method for hardening first transparent layer 21 being a clear epoxy and then hardening second transparent layer 22 being also a clear epoxy is adopted.

A very thin air layer not shown in Fig. 8 is formed at an interface 200 between first and second transparent layers 21 and 22 in the step of forming second transparent layer 22 on first transparent layer 21.

An operation will now be described.

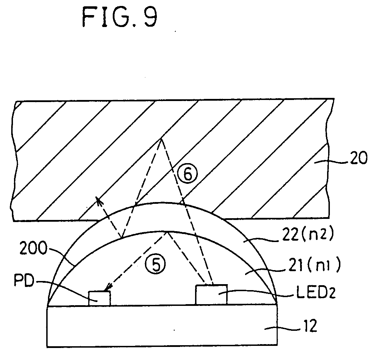

Referring to Fig. 9, the light emitting diode for a photosensor is made contact with a living body 20. Since refractive index n$_1$ of first transparent layer 21 is larger than that n$_2$ of second transparent layer 22, a portion of the light emitted from LED chips LED$_1$ and LED$_2$ undergoes a total reflection at interface 200 and reaches photodiode PD, as shown by the dotted line ⑤. In the embodiment, since the amount of the light subjected to the total reflection increases, the amount of the light emitted from the LED chips, which is directly received by photodiode PD, increases.

The light scattered or reflected by living body 20 shown by the dotted line ⑥, of the light which is emitted from LED chips LED$_1$ and LED$_2$ and is then incident on living body 20, undergoes a total reflection at interface 200 between first and second transparent layers 21 and 22 and thus does not reach photodiode PD.

As has been mentioned above, since the light emitting diode for a photosensor according to the present invention is thus structured, the amount of the light emitted from the light emitting element, which is directly received by the photodiode, increases, thereby enabling a neglect of the amount of the light reflected from the living body. This makes it possible to retain a constant amount of light and make a precise feedback, thereby enabling a precise measurement under no influence caused by a drift of temperatures.

In addition, a photosensor including the above-described light emitting diode enables a precise measurement immune to influence caused by a drift of temperatures.

Although the present invention has been described and illustrated in detail, it is clearly understood that the same is by way of illustration and example only and is not to be taken by way of limitation, the spirit and scope of the present invention being limited only by the terms of the appended claims.

**Claims**

1. A light emitting diode for a photosensor, comprising:

a substrate (12);

a light emitting element (LED$_2$) provided on said substrate (12);

a light receiving element (PD) disposed on said substrate (12) and in the vicinity of said light emitting element (LED$_2$), for receiving light emitted from said light emitting element (LED$_2$), to detect a change in the amount of the light from said light emitting element (LED$_2$) in accordance with a change in ambient temperature;

a first transparent layer (21) provided on said substrate (12) to cover said light emitting element (LED$_2$) and said light receiving element (PD); and

a second transparent layer (22) provided on said first transparent layer (21).

2. The light emitting diode according to claim 1, wherein

an air layer is interposed between said first transparent layer (21) and said second transparent layer (22).

3. The light emitting diode according to claim 2, wherein

said air layer has a thickness larger than a wavelength of the light emitted from said light emitting element (LED$_2$).

4. The light emitting diode according to claim 1, wherein

an interface between said first and second transparent layers (21, 22) is hemispherical.

5. The light emitting diode according to claim 4, wherein

a surface of said second transparent layer (22) is hemispherical.

6. The light emitting diode according to claim 1, wherein

said first transparent layer (21) is formed of a material for intercepting light other than a wavelength of a spectrum of the light emitted from said light emitting element (LED$_2$).

7. The light emitting diode according to claim 1, wherein

said first transparent layer (21) is formed of a transparent body of the type which limits a wavelength band region.

8. The light emitting diode according to claim 7, wherein

said transparent body for limiting the wavelength band region includes an epoxy resin.

9. A light emitting diode for a photosensor, comprising:

a substrate (12);

a light emitting element (LED$_2$) provided on said substrate (12);

a light receiving element (PD) disposed on said substrate (12) and in the vicinity of said light emitting element (LED$_2$) for receiving light emitted from said light emitting element (LED$_2$), to detect a change in the amount of the light from said light emitting element (LED$_2$) in accordance with a change in ambient temperature;

a first transparent layer (21) provided on said substrate (12) to cover said light emitting element (LED$_2$) and said light receiving element (PD); and

a second transparent layer (22) provided on said first transparent layer (21), wherein

a refractive index $n_1$ of said first transparent layer (21) and a refractive index $n_2$ of said second transparent layer (22) satisfy an inequality, $n_1 \geq n_2$.

10. A photosensor including the light emitting diode for a photosensor recited in claim 1.

11. A photosensor including the light emitting diode for a photosensor recited in claim 9.

FIG.1

FIG.2

FIG. 3A

FIG. 3B

FIG. 4

FIG. 5

FIG. 6

## FIG. 7

## FIG. 8

FIG. 9